# EUROPÄISCHE PATENTSCHRIFT

(11) **EP 1 011 854 B1**
(45) Veröffentlichungstag und Bekanntmachung des Hinweises auf die Patenterteilung: **02.04.2003**
(21) Anmeldenummer: 99934558.0
(22) Anmeldetag: 02.07.1999
(51) Int. Cl.: B01F 7/00, B01F 7/16

(54) **VERFAHREN ZUR HERSTELLUNG VON WÄSSRIGEN EMULSIONEN ODER SUSPENSIONEN**
METHOD FOR PRODUCING AQUEOUS EMULSIONS OR SUSPENSIONS
PROCEDE POUR LA PREPARATION D'EMULSIONS OU DE SUSPENSIONS AQUEUSES

(30) Priorität: 02.07.1998 DE 19829647
(43) Veröffentlichungstag der Anmeldung: 28.06.2000
(73) Patentinhaber: Wella Aktiengesellschaft, 64274 Darmstadt (DE)
(72) Erfinder: HUFF, Rainer, D-36166 Hünfeld (DE); FRAUENRIEDER, Gerd, D-36151 Burghaun (DE); BIEBER, Bernhard, D-36088 Hünfeld (DE)
(86) Internationale Anmeldenummer: EP9904589
(87) Internationale Veröffentlichungsnummer: WO00001474

(56) Entgegenhaltungen:
- WO-A-94/13395
- WO-A-96/22830
- DE-A- 2 004 143
- DE-A- 2 931 782
- DE-U- 8 230 048
- GB-A- 405 503
- US-A- 2 641 453
- US-A- 5 366 288

## Beschreibung

Gegenstand der Erfindung ist ein Verfahren zur Herstellung von wäßrigen Emulsionen oder Suspensionen.

Disperse Zubereitungen vom Typ der Emulsionen oder Suspensionen spielen bei der Kosmetik-, Pharma- und Lebensmittelherstellung eine herausragende Rolle. Daher kommt der Optimierung des Herstellungsverfahrens insbesondere hinsichtlich einer Zeit- und Energieeinsparung eine besondere Bedeutung zu. Eine Übersicht über moderne Verfahren zur Herstellung von halbfesten und flüssigen Emulsionen ist dem Artikel im SÖFW-Journal, 124. Jahrgang, 5/98, Seiten 308 bis 313 sowie dem Artikel im SÖFW-Journal, 118. Jahrgang, 5/92, Seiten 287 bis 296 zu entnehmen. Die Verfahren können hinsichtlich der Temperaturführung in heiß/heiß-, heiß/kalt- und kalt/kalt-Verfahren unterschieden werden.

Das Standardverfahren zur Emulsionsherstellung ist das heiß/heiß-Verfahren, bei dem die Fettphase auf ca. 75°C erhitzt und mit der ebenfalls ca. 75°C heißen Wasserphase vereinigt wird. Anschließend erfolgt mit hohem Zeitaufwand die Entfernung der zuvor in Form von Wärme zugeführten überschüssigen Energie. Daher ist dieses Verfahren sehr zeit- und kostenintensiv.

Um sowohl den Energieverbrauch als auch die Herstellungszeit zu verringern, sind die sogenannten heiß/kalt- und kalt/kalt-Verfahren entwickelt worden.

Diese Verfahren haben jedoch den Nachteil, daß sie an bestimmte Voraussetzungen gebunden sind und daher ihre Anwendbarkeit nur sehr begrenzt ist und nicht für jede beliebige Emulsion oder Dispersion brauchbar sind. Bei dem heiß/kalt-Verfahren wird beispielsweise die heiße Ölphase vorgelegt und nicht erwärmtes Wasser einhomogenisiert. Voraussetzungen hierfür waren bisher eine sehr langsame Wasserzugabe zur Vermeidung von Kristallisationen durch Schockkühlung sowie ein genügend hoher Fettanteil zur Verhinderung eines Unterschreitens des Erstarrungspunktes während der Wasserzugabe. Dieses Verfahren ist daher immer noch zeitintensiv und zudem auf fettreiche Emulsionen beschränkt.

Aus der WO 95/13787 ist bekannt, daß es zur Emulsionsherstellung möglich ist, Fettphase und wäßrige Phase kalt zu vermischen, allerdings nur unter der Voraussetzung, daß erstens ein geeigneter Emulgator anwesend ist und daß es sich zweitens bei der Fettphase um ein mittelpolares Öl handelt. Zur Kaltemulgierung von Ölkörpern mit hoher oder niedriger Polarität ist es erforderlich, daß zusätzlich Metallseifen anwesend sind. Diese Metallseifen müssen zunächst bei erhöhten Temperaturen in Öl gelöst und anschließend abgekühlt werden, was ebenfalls zeit- und kostenintensiv ist.

Nach herkömmlichen Verfahren hergestellte Emulsionen oder Suspensionen weisen auch häufig den Nachteil auf, daß die emulgierten oder suspendierten Teilchen eine zu uneinheitliche oder zu große Teilchengröße und damit verbunden eine für viele Anwendungen zu geringe wirksame Oberfläche aufweisen und zudem in der Emulsion bzw. Suspension nicht optimal verteilt sind.

In der US-A-2 641 453 werden Mischer zur Herstellung von Dispersionen von Feststoffen in Flüssigkeiten (slurries of cementitious materials) beschrieben. Die Zusammenführung der zu vermischenden Phasen erfolgt vor den Zahnkränzen. In der WO 94 13395 A und in der WO 96 22830 A werden Verfahren zur Injektion eines ersten Fluids in ein zweites Fluid beschrieben, wobei es sich gemäß den Beispielen um die Injektion von Gasen in Flüssigkeiten handelt. In der DE 82 30 048 U werden Mischer zur Einführung von Luft in Flüssigkeiten beschrieben, insbesondere zur Herstellung von Soßen und Teigen, welche extrem aufgeschlagen und luftig sind. In der DE 29 31 782 A wird ein Verfahren zum Mischen von flüssigen mit festen Bestandteilen beschrieben. In der GB 405 503 A werden spezielle Mischapparaturen (Rührkessel) u.a. zur Herstellung von Emulsionen beschrieben. In der DE 20 04 143 A wird eine Vorrichtung zur Herstellung von Emulsionen bzw. Suspensionen beschrieben, wobei die Zusammenführung der zu vermischenden Phasen bereits vor den Zahnkränzen erfolgt. In der US-A-5 366 288 wird eine Vorrichtung zur Suspendierung eines faserförmigen Feststoffs (fibrous material) in einer Flüssigkeit beschrieben.

Es bestand somit die Aufgabe, ein vereinfachtes und breit anwendbares Verfahren zur Herstellung von Suspensionen oder Emulsionen zur Verfügung zu stellen, welches zeit- und kostengünstig ist, gleichzeitig den oben genannten Beschränkungen nicht unterliegt sowie die gewünschten Eigenschaften der hergestellten Produkte nicht wesentlich beeinträchtigt oder sogar noch verbessert.

Gelöst wird die Aufgabe durch ein Verfahren zur Herstellung von wäßrigen Emulsionen oder Suspensionen
wobei
(A) in einem Behälter eine wäßrige Phase vorgelegt wird, wobei an den Behälter ein Homogenisator vom Rotor/Stator-Typ angeschlossen ist und der Homogenisator einen zusätzlichen Anschluß aufweist, durch den eine einzuhomogenisierende Phase direkt auf den Rotor aufgegeben werden kann und diese Phase erst in den Zahnkränzen des Homogenisators mit der vorgelegten Phase in Berührung kommt
(B) der Homogenisator gestartet wird und danach
(C) eine einzuhomogenisierende, in der vorgelegten Phase unlösliche zweite Phase, welche bei Raumtemperatur fest vorliegt, in geschmolzenem Zustand über den zusätzlichen Anschluß des Homogenisators zugeführt wird,
wobei die wäßrige Phase nicht erwärmt ist.

Der Begriff 'wäßrige Phase' umfaßt Wasser sowie Gemische von Wasser mit wasserlöslichen Lösungsmitteln wie niederen Alkoholen, z.B. Ethanol oder Isopropanol oder Polyolen wie Ethylenglykol, Diethylenglykol, Butylenglykol oder Glycerin.

Die einhomogenisierten Teilchen weisen eine optimale Teilchengröße von beispielsweise rund 1 µm, eine optimale Verteilung in der externen Phase und eine hohe wirksame Oberfläche auf. Hierdurch bedingt lassen sich Rohstoffeinsparungen realisieren, da die einzuhomogenisierende Substanz in geringeren Mengen eingesetzt werden kann. Auch stellt sich die Problematik der unerwünschten Kristallisationsvorgänge nicht.

Liegt die einzuhomogenisierende Phase bei Raumtemperatur in fester Form vor, so wird sie in geschmolzenem Zustand zugegeben. Hierbei kann es sich um Wachse oder um wachsartige Stoffe handeln, beispielsweise um natürliche, nachwachsende Wachse (Insekten-, Tier- und Pflanzenwachse), fossile Wachse (Erdöl-, Braunkohlen-, Torfwachse oder Ozokerite), synthetische Wachse (Fischer-Tropsch-, Polyethylen- oder Amidwachse) höherschmelzende Paraffine, Ester, Fette, langkettige Carbonsäuren oder langkettige Alkohole, jeweils mit Schmelz- bzw. Erstarrungspunkten oberhalb Raumtemperatur.

Die Temperaturen von vorgelegter Phase und zugeführter Schmelze werden idealerweise so gewählt, daß die resultierende Mischtemperatur unterhalb des Kristallisations- oder Erstarrungspunktes der einzuhomogenisierenden Substanz liegt. Beträgt die Temperatur des geschmolzenen Wachses beispielsweise 70 bis 90 °C, so kann mit einer Temperatur der vorgelegten wässrigen Phase von 10 bis 25 °C eine Mischtemperatur erreicht werden, die zwischen 20 und 40°C liegt. Die erhaltene Wachssuspension kann dann unmittelbar anschließend gebrauchsfertig abgefüllt werden, ohne daß ein langwieriges Abkühlen erforderlich ist und ohne daß die Gefahr einer nachträglichen, zeitverzögerten Viskositäts- oder Konsistenzänderung besteht, da die Kristallisationseffekte sofort abgeschlossen sind.

In einer besonderen Ausführungsform wird die einzuhomogenisierende Phase, insbesondere ein geschmolzenes Wachs oder eine Schmelze einer Substanz von bei Raumtemperatur wachsartiger Konsistenz ohne Emulgator einhomogenisiert.

Weiterhin als einzuhomogenisierende Substanz geeignet sind Mono- oder Diester der Formeln R¹-COOR², R¹-COO-R³-OOCR¹ und R²OOC-R³-COOR², wobei R¹ für eine C8-bis C22-Alkylgruppe, R² für eine C3- bis C22-Alkylgruppe und R³ für eine C2- bis C16-Alkylengruppe steht. Geeignet sind auch natürlich vorkommende Monoester- bzw. Wachsestergemische, wie sie z.B. in Jojobaöl oder Spermöl vorliegen und verzweigte primäre Alkohole, wie sie unter der Bezeichnung Guerbetalkohole bekannt sind.

Als einzuhomogenisierende Substanzen sind außerdem Stoffe geeignet, die üblicherweise als Trübungsmittel in kosmetischen Mitteln eingesetzt werden, insbesondere solche der Formel R¹-COO-(CHR⁴CHR⁵O)ₙ-COR⁶, wobei R¹ für eine C8-C22-Alkylgruppe, R⁴ und R⁵ für Wasserstoff oder Methyl und R⁶ für Wasserstoff oder für R¹ steht und n eine Zahl zwischen 1 und 12, vorzugsweise 1, 2, 3 oder 4 bedeutet. Bevorzugt sind Glykoldifettsäureester.

Der Anteil der einzuhomogenisierenden Substanz an der fertigen Emulsion oder Suspension richtet sich nach den Anforderungen des herzustellenden Endproduktes. Er kann für Haarkuren beispielsweise von 2 bis 10 Gewichtsprozent oder für Cremes wie z.B. Haarfärbecremes auch bis ca. 50 Gewichtsprozent betragen.

Ein wesentlicher Bestandteil des Verfahrens ist der einzusetzende Homogenisator. Hierbei handelt es sich um einen Homogenisator vom Rotor/Stator-Typ, der einen zusätzlichen Anschluß in einer ganz bestimmten Position aufweist. Herkömmliche Rotor/Stator-Homogenisatoren werden beispielsweise beschrieben in Andreas Domsch, 'Die kosmetischen Präparate', Band III, 4. Auflage, Seite 305 bis 307 sowie in dem Artikel in Seifen-Öle-Fette-Wachse, 112. Jahrgang, 1986, Seiten 532 bis 536. Das Rotor-Stator-Prinzip ist das am häufigsten angewendete Homogenisierverfahren bei der Herstellung kosmetischer Präparate. Hierbei wird das zu homogenisierende Gut mittels eines sich drehenden Teils, dem Rotor, durch einen feststehenden Teil, den Stator, hindurchbewegt, wobei zwischen dem Rotor und dem Stator nur ein äußerst geringer Spalt besteht. Die Homogenisierwirkung beruht auf den im Scherspalt auftretenden Turbulenzen. Bei Verwendung von herkömmlichen Homogenisatoren ohne einen zusätzlichen Anschluß ist die einzuhomogenisierende Substanz bereits in Kontakt mit der wässrigen Phase, liegt also als Vormischung oder Voremulsion vor. Mit diesen herkömmlichen Homogenisatoren sind Emulsionen nur nach den herkömmlichen Verfahren, nicht jedoch nach dem erfindungsgemäßen Verfahren herstellbar. Nach dem erfindungsgemäßen Verfahren ist ein Kontakt der einzuhomogenisierenden Substanz mit der vorgelegten Phase vor dem eigentlichen Homogenisiervorgang zu vermeiden.

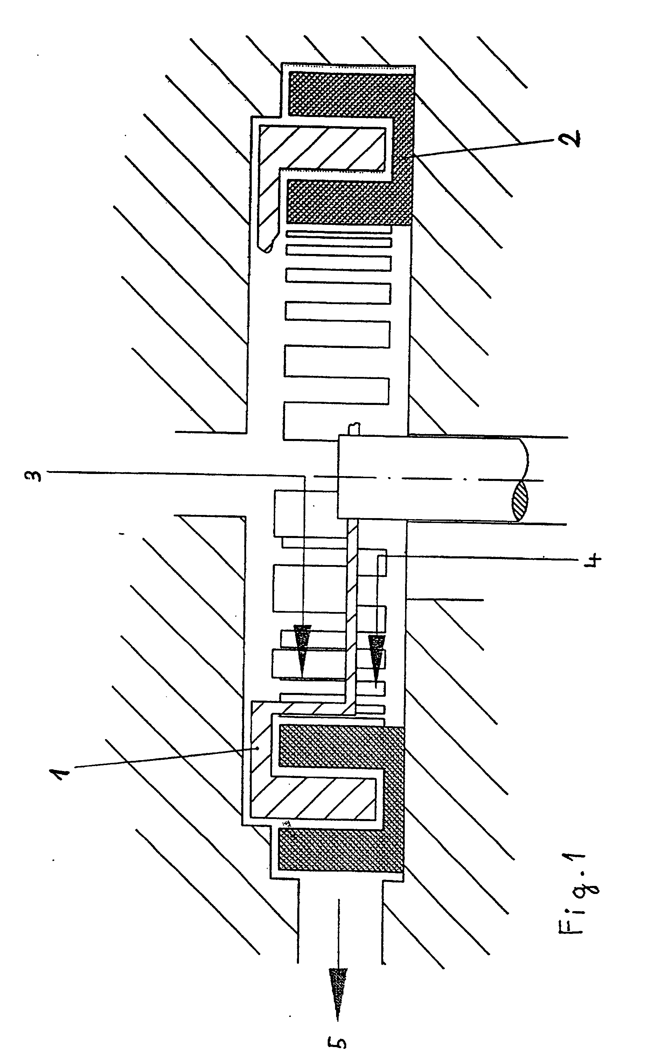

Ein geeigneter Homogenisator mit Rotor (1) und Stator (2) ist in Figur 1 dargestellt. Die vorgelegte Phase (3) kommt erst in den Zahnkränzen mit der einzuhomogenisierenden, zweiten Phase (4) in Berührung. Das Fertigprodukt (5) tritt in Form einer feinen Dispersion aus.

Ein weiterer Gegenstand der Erfindung ist die Verwendung eines Homogenisators vom Rotor/Stator-Typ welcher einen zusätzlichen Anschluß aufweist, durch den eine einzuhomogenisierende Substanz direkt auf den Rotor aufgegeben werden kann und die Substanz erst in den Zahnkränzen des Homogenisators mit der externen, wässrigen Phase in Berührung kommt, zur Herstellung von Suspensionen aus geschmolzenem Wachs oder geschmolzenen wachsartigen Stoffen und nicht erwärmtem Wasser. Insbesondere können Lotionen, Cremes, Salben, emulsionsförmige kosmetische Mittel, emulsionsförmige Lebensmittel oder emulsionsförmige pharmazeutische Mittel vorteilhaft hergestellt werden. Emulsionsförmige kosmetische Mittel sind z.B. Haar- oder Hautbehandlungsmittel wie Haarpflegemittel, Haarkurmittel, Haarfärbemittel, Hautcremes oder Sonnencremes. Emulsionsförmige Lebensmittel sind z.B. Soßen, Mayonnaisen, cremige Brotaufstriche, emulsionsförmige Salatdressings oder Margarine.

Es wurden Versuche zur Kaltemulgierung durchgeführt, bei denen die einzuhomogenisierende Substanz über einen zusätzlichen Anschluß in den Zufluß zum Homogenisator eingespeist wurde, wobei der zusätzliche Anschluß unmittelbar vor der Rotor/Stator-Einheit positioniert wurde. Diese Versuche führten nicht zu einem befriedigenden Ergebnis, da hier immer noch ein, wenn auch nur sehr kurzzeitiger, Kontakt zwischen einzuhomogenisierender Substanz und äußerer Phase vor dem eigentlichen Homogenisiervorgang vorliegt. Vermutlich kommt es zu Ausgeelierungen, Klumpenbildungen oder Ausfällungen, die zumindest eine weniger feine Emulsion oder eine längere Ansatzzeit bewirken und im Extremfall sogar zum Blockieren des Rotors führen können. Erfindungsgemäß muß der Homogenisator daher über einen zusätzlichen Anschluß verfügen, der so positioniert ist, daß die einzuhomogenisierende Substanz direkt in die Rotor/Stator-Einheit gelangt, ohne zuvor mit der übrigen äußeren Phase in Kontakt gekommen zu sein. Im Prinzip kann jeder handelsübliche Homogenisator verwendet werden, sofern er konstruktiv in der beschriebenen Weise verändert ist. Ein besonders geeigneter Homogenisator wird beispielsweise von der Firma Berents unter der Bezeichnung Becomix Duo-Homogenisator DH 2000 angeboten.

Das Verhältnis der dem Homogenisator zufließenden Teilströme von vorgelegter Phase einerseits und einzuhomogenisierender Phase andererseits werden idealerweise so eingestellt, daß die über den zusätzlichen Anschluß zugeführte einzuhomogenisierende Phase nicht in einen den Zahnkränzen der Rotor/Statoreinheit vorgelagerten Bereich transportiert wird, wo sie vor Homogenisierung mit der vorgelegten Phase in Kontakt kommen kann. Die Einstellung des die einzuhomogenisierende Phase zuführenden Teilstroms kann durch Anlegen eines Druckunterschieds erfolgen. Vorzugsweise wird ein leichter Unterdruck von beispielsweise 0,2 bis 0,8 bar, vorzugsweise 0,3 bis 0,6 bar angelegt. Die Einstellung des die vorgelegte Phase zuführenden Teilstroms kann durch Einstellung der Umfangsgeschwindigkeit des Rotors erfolgen. Bei typischen, handelsüblichen Rotoren beträgt die Umfangsgeschwindigkeit beispielsweise 20 bis 40 m/s, vorzugsweise 25 bis 30 m/s.

Die Homogenisierung kann emulgatorfrei erfolgen. Es kann aber auch ein Emulgator oder ein Tensid als Emulgierhilfe anwesend sein, welches vorzugsweise vor Zuführung der einzuhomogenisierenden Substanz über den zusätzlichen Anschluß des Homogenisators eingebracht wurde. Der Emulgator kann in Mengen von 0,5 bis 30 Gewichtsprozent der fertigen Zusammensetzung vorliegen.

Als Emulgatoren sind nicht-ionische, anionische, kationische, amphotere oder zwitterionische Emulgatoren geeignet. Geeignete Emulgatoren sind beispielsweise die im 'International Cosmetic Ingredient Dictionary and Handbook', 7.Auflage, Band 2 im Abschnitt 'Surfactants', insbesondere im Unterabschnitt 'Surfactants-Emulsifying Agents' aufgeführten Emulgatoren.

Nichtionische Emulgatoren sind beispielsweise oxethylierte Fettalkohole, oxethylierte Nonylphenole, Fettsäuremono- und diglyceride, ethoxyliertes und hydriertes oder nicht hydriertes Rizinusöl, Fettsäurealkanolamide, oxethylierte Fettsäureester. Kationische Emulgatoren sind beispielsweise langkettige quaternäre Ammoniumverbindungen wie sie unter den CTFA-Bezeichnungen 'Quaternium' bekannt sind wie z.B. Alkyltrimethylammoniumsalze oder Dialkyldimethylammoniumsalze mit C8- bis C22-Alkylgruppen. Anionische Emulgatoren sind beispielsweise Fettalkoholsulfate, Alkylethersulfate, Alkylbenzolsulfonate. Amphotere Emulgatoren sind beispielsweise die verschiedenen bekannten Betaine wie Fettsäureamidoalkylbetaine und Sulfobetaine und C8- bis C22-Alkylbetaine.

Das erfindungsgemäße Verfahren ist besonders geeignet zur Herstellung einer Trübungsmittelzusammensetzung für kosmetische Mittel. Hierbei wird zunächst ein konzentriertes Alkylethersulfat, beispielsweise Laurylethersulfat über den Homogenisator in vorgelegtem, nicht erwärmtem, elektrolytfreiem Wasser gelöst. Anschließend wird ein wasserunlösliches Trübungsmittel, beispielsweise ein Ethylen- oder Polyethylenglykoldifettsäureester wie Polyethylenglykol-(3)-distearat in geschmolzenem Zustand einhomogenisiert.

Das erfindungsgemäße Verfahren eignet sich insbesondere zur Herstellung von kosmetischen und pharmazeutischen Mitteln, wobei entweder die kosmetischen Wirk- und Zusatzstoffe in der vorgelegten Phase enthalten sind und danach die Herstellung der Emulsion oder der Suspension nach dem erfindungsgemäßen Verfahren erfolgt, oder wobei zunächst die Emulsion oder Suspension hergestellt wird und anschließend die kosmetischen oder pharmazeutischen Wirk- und Zusatzstoffe eingebracht werden. Da die erfindungsgemäß primär hergestellte Emulsion oder Suspension äußerst fein und niedrigviskos ist, lassen sich die weiteren Wirk- und Zusatzstoffe wesentlich einfacher und schneller einarbeiten als nach herkömmlichen Verfahren, wodurch die Ansatzzeiten deutlich reduziert werden.

Von besonderem Vorteil ist auch die erfindungsgemäße Herstellung von Haarfärbecremes, insbesondere für Oxidationshaarfarben mit einem Gehalt an oxidationsempfindlichen Farbstoffvorprodukten. Diese Haarfärbecremes beruhen üblicherweise auf einer wachshaltigen Cremebasis. Bei herkömmlichen Herstellungsverfahren wird eine auf ca. 70 bis 80 °C erwärmte hydrophobe, geschmolzene Wachsphase bei einer Temperatur von ca. 70 bis 80 °C in eine wäßrige, die Farbstoffvorprodukte sowie gegebenenfalls weitere übliche Zusatzstoffe enthaltende wäßrige Phase einemulgiert. Anschließend muß sehr langsam und verzögert unter Rühren abgekühlt werden, um ein unkontrolliertes Auskristallisieren des Wachses (Bildung von Wachsstippen) zu verhindern. Bei diesem relativ lang dauerndem Abkühlen kann es durch Einrühren von Luftsauerstoff zu unerwünschten Oxidationsreaktionen kommen. Außerdem kann es zu einem Nachverdicken kommen, was Schwierigkeiten bei der Abfüllung des Fertigproduktes bereiten kann.

Die Nachteile des herkömmlichen Verfahrens der Heißemulgierung werden durch das erfindungsgemäße Verfahren überwunden. Es ist kein zeit- und energieverbrauchendes Aufheizen und Abkühlen der gesamten Masse erforderlich. Die Gefahr der Stippenbildung und von unerwünschten Oxidationsreaktionen ist deutlich reduziert. Die erhaltene Färbemasse kann unmitttelbar nach Zuführung der Wachsphase ohne weitere Homogenisierungsschritte als Fertigprodukt abgefüllt werden, da weder ein Abkühlen erforderlich ist, noch eine Änderung der Konsistenz durch Nachverdicken erfolgt. Aufgrund der wesentlich feineren Verteilung der hydrophoben Phase wird eine wesentlich größere spezifische Oberfläche und damit eine höhere Wirksamkeit der Rohstoffe erreicht. Dies hat zur Folge, daß deutlich weniger Rohstoffe eingesetzt werden können, was zu Mengenersparnissen von ca. 10 bis 30% gegenüber auf herkömmlichem Wege hergestellten Crememassen führt.

Gegenstand der Erfindung ist daher insbesondere ein Verfahren zur Herstellung einer Haarfärbecreme, wobei
(A) in einem Behälter Haarfarbstoffe bzw. Farbstoffvorprodukte und gegebenenfalls weitere übliche Zusatzstoffe in nicht erwärmtem Wasser vorgelegt werden, wobei an den Behälter ein Homogenisator vom Rotor/Stator-Typ angeschlossen ist und der Homogenisator einen zusätzlichen Anschluß aufweist, durch den eine einzuhomogenisierende Phase direkt auf den Rotor aufgegeben werden kann und diese Phase erst in den Zahnkränzen des Homogenisators mit der vorgelegten Phase in Berührung kommt
(B) der Homogenisator gestartet wird,
(C) eine Schmelze einer bei Raumtemperatur wachsartigen Substanz mit gegebenenfalls darin gelösten weiteren, üblichen hydrophoben Zusatzstoffen über den zusätzlichen Anschluß des Homogenisators zugeführt und dadurch in die vorgelegte wäßrige Phase einhomogenisiert wird.

Idealerweise wird die Temperatur der vorgelegten wäßrigen Phase so gewählt, daß die nach Einemulgieren der Wachsschmelze resultierende Mischtemperatur unterhalb des Erstarrungspunktes des Wachses liegt. Beträgt die Temperatur des geschmolzenen Wachses beispielsweise 70 bis 90 °C, so kann mit einer Temperatur der vorgelegten wäßrigen Phase von 10 bis 25 °C eine Mischtemperatur erreicht werden, die zwischen 20 und 40°C liegt. Die Färbecreme kann dann unmittelbar anschließend gebrauchsfertig abgefüllt werden.

Soll es sich bei dem Endprodukt um eine viskose Zusammensetzung handeln, so ergibt sich die gewünschte Endviskosität, insbesondere, wenn es sich um O/W-Emulsionen handelt, häufig bereits durch das Homogenisieren. Die gewünschte Endviskosität kann aber auch (vorzugsweise zum Abschluß des Herstellungsverfahrens) durch Zugabe eines Elektrolyten wie Natriumchlorid oder eines anderen, verdickend wirkenden Stoffes wie Cellulosen oder Cellulosederivaten eingestellt werden.

Nach herkömmlichen Verfahren hergestellte Zusammensetzungen zeigen häufig den Effekt des Nachverdickens, das heißt die engültige Viskosität und Konsistenz stellt sich erst nach einer gewissen Lagerungszeit ein. Nach dem erfindungsgemäßen Verfahren hergestellte Zusammensetzungen weisen diesen Nachteil nicht auf sondern besitzen bereits direkt nach Abschluß des Herstellverfahrens ihre endgültige Viskosität.

Nach herkömmlichen Verfahren hergestellte Zusammensetzungen zeigen häufig den Effekt einer Viskositätsänderung unter Druck bzw. bei oder nach mechanischer Beanspruchung, was sich insbesondere beim Abfüllen der Produkte mit Pumpen negativ bemerkbar macht oder ein Umfüllen mit üblichen Pumpen sogar verhindern kann. Nach dem erfindungsgemäßen Verfahren hergestellte Zusammensetzungen weisen diesen Nachteil nicht auf sondern sind druckunempfindlich und können problemlos mit üblichen Pumpen gefördert und abgefüllt werden.

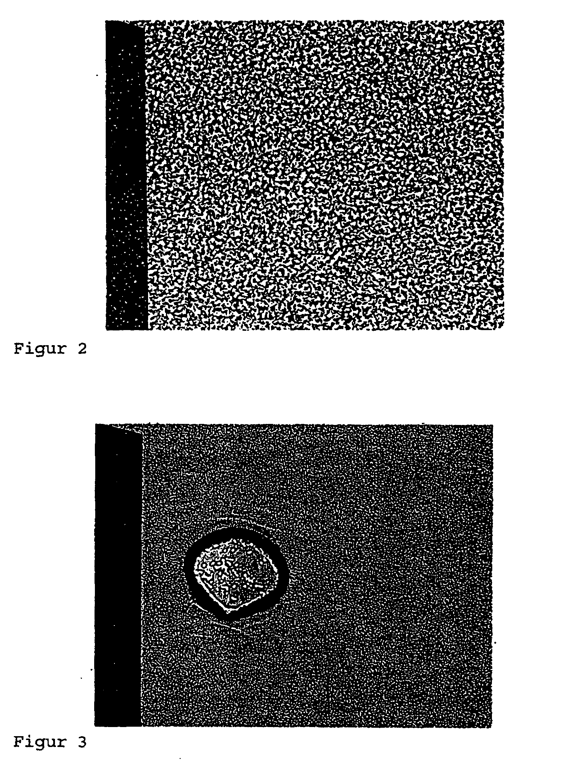

Es hat sich gezeigt, daß erfindungsgemäß hergestellte Emulsionen oder Suspensionen gegenüber nicht erfindungsgemäß, auf herkömmlichem Wege hergestellten Suspensionen oder Emulsionen bei gleicher Konzentration und Menge an hydrophoben Stoffen vorteilhafte Eigenschaften aufweisen. Physikalisch macht sich dies in einer höheren Viskosität der erfindungsgemäß hergestellten Zusammensetzungen bemerkbar. Aber auch die anwendungstechnischen Eigenschaften der Produkte werden verbessert. So wurde für ein Haarpflegemittel in Form eines Pumpschaumes gefunden, daß Griff und Kämmbarkeit des behandelten Haares besser sind und der Schaum cremiger und weicher ist als bei einem nicht erfindungsgemäß hergestelltem Produkt mit identischer chemischer Zusammensetzung. Die verbesserten Konsistenz- und anwendungstechnischen Eigenschaften sind vermutlich auf eine optimalere, feinere Verteilung der internen Phase, bzw. auf einen geringeren Teilchendurchmeser der dispergierten Phase zurückzuführen, wie ein Vergleich der Figuren 2 und 3 zeigt. Figur 3 zeigt eine gemäß Beispiel 1 erfindungsgemäß hergestellte Dispersion. Figur 2 zeigt eine nicht erfindungsgemäß hergestellte Dispersion gleicher chemischer Zusammensetzung aber deutlich feinerer und gleichmäßigerer Verteilung der dispergierten Phase. Gegenstand der Erfindung sind daher auch wäßrige Emulsionen oder Suspensionen, welche nach dem erfindungsgemäßen Verfahren hergestellt sind. Der Teilchendurchmesser der dispergierten Phase ist vorzugsweise kleiner 1 µm, besonders bevorzugt kleiner 0,2 µm.

Der Begriff 'nicht erwärmt' im Sinne dieser Anmeldung bedeutet, daß ein Stoff oder ein Stoffgemisch nicht durch Zufuhr von Heizenergie auf eine Temperatur oberhalb der Umgebungstemperatur gebracht wurde sondern bei der Umgebungstemperatur oder darunter vorliegt.

### Beispiele

### Beispiel 1: Herstellung eines Trübungsmittelkonzentrats

113 Liter Wasser und 450 g Zitronensäure wurden bei einer Temperatur von 13,5 °C in einem Kessel vorgelegt, an den ein Homogenisator vom Typ Becomix Duo-Homogenisator DH 2000 (Berents GmbH & Co. KG) angeschlossen war. Mit Hilfe einer Pumpe wurden 18 kg 70 %iges Laurylethersulfat über den zusätzlichen Anschluß des Homogenisators in das Wasser eingetragen, wobei die Temperatur 15°C betrug. Anschließend wurde eine 86°C warme Schmelze von Polyethylenglykol-(3)-distearat über den zusätzlichen Anschluß des Homogenisators in die wäßrige Tensidlösung eingetragen. Die Mischtemperatur nach dem Eintragen betrug 27,5 °C. Weitere 18 kg 70%iges Laurylethersulfat wurden in zwei Schritten über den zusätzlichen Anschluß des Homogenisators einhomogenisiert. Es ist aber auch möglich, die gesamte Tensidmenge gleich zu Beginn in das Wasser einzuarbeiten. Die Endtemperatur der Emulsion betrug 36°C.

Die erhaltene Masse ist dünnflüssig (Viskosität 140 mPa nach MV-DIN/7) und sehr homogen. Die Masse wurde einem Belastungstest in einer Zentrifuge bei 2000 U/min unterzogen. Nach einer Belastung von 5 Minuten zeigt die Masse keinerlei Separation. Figur 3 zeigt eine vergrößerte, fotografische Darstellung der erhaltenen Dispersion. (Die Kreisform ist ein Einschluß eines Luftbläschens.) Die Größe der dispergierten Teilchen ist kleiner oder gleich 0,2 µm.

Die Einstellung der Endviskosität erfolgte mit Kochsalz. Die erhaltene Zusammensetzung eignet sich für eine Verwendung als Trübungsmittel bei der Herstellung von Shampoos.

### Vergleichsbeispiel 1: Herkömmlich hergestelltes Trübungsmittelkonzentrat

Figur 2 zeigt eine vergrößerte fotografische Darstellung einer auf herkömmlichem Wege hergestellten Dispersion gleicher chemischer Zusammensetzung wie in Beispiel 1. Die Vergrößerung ist die gleiche wie bei der erfindungsgemäßen Dispersion gemäß Figur 3. Die Größe der dispergierten Teilchen ist größer oder gleich 1 µm.

### Beispiel 2: Herstellung einer emulgatorhaltigen Wachssuspension in Wasser

95 kg Wasser und 8 kg einer 25%igen Lösung von Cetyltrimethylammoniumchlorid wurden bei einer Temperatur von 12 °C in einem Kessel vorgelegt, an den ein Homogenisator vom Typ Becomix Duo-Homogenisator DH 2000 (Berents GmbH & Co. KG) angeschlossen war. Über den zusätzlichen Anschluß des Homogenisators wurden 5 kg flüssiger, auf 65°C erwärmter Cetearylalkohol in das vorgelegte Wasser eingetragen, wobei ein Vakuum von 0,4 bar angelegt wurde. Anschließend wurde noch für 5 Minuten im Kreislauf homogenisiert. Es entstand eine homogene, hochviskose Masse.

Ein Vergleich mit einer auf herkömmliche Weise hergestellten Emulsion zeigt, daß bei dem erfindungsgemäßen Verfahren durch die hohe Verteilung der Rohstoffe Rohstoffeinsparungen von mindestens 50% möglich sind. Eine Verdünnung der erfindungsgemäß hergestellten Masse mit Wasser im Verhältnis 1:1 liefert eine brauchbare, homogene Emulsion mit einer Viskosität von 520 mPas bei 25 °C.

### Beispiel 3: Herstellung einer Creme-Grundmasse

106 kg Wasser wurden kalt (19 °C) in einem Rührkessel vorgelegt, an den ein Homogenisator vom Typ Becomix Duo-Homogenisator DH 2000 (Berents GmbH & Co. KG) angeschlossen war. Über den zusätzlichen Anschluß des Homogenisators wurden 8 kg 70%iges Natriumlaurylethersulfat in das vorgelegte Wasser eingetragen, wobei ein Vakuum angelegt wurde. 11,1 kg einer 25%igen wäßrigen Lösung von Alkylsulfaten (Texapon KE 2501 der Firma Henkel KGaA) wurden von oben unter Rühren zu dem Ansatz gegeben. Eine Mischung aus 10,12 kg Glycerylstearat, 3,68 kg Lanolinalkohol, 4 kg Glykoldistearat und 28,98 kg Cetearylalkohol wurde bei 85 °C aufgeschmolzen und über den zusätzlichen Anschluß des Homogenisators eingetragen, wobei ein Vakuum angelegt wurde. Es entstand eine homogene, viskose Creme.

### Beispiel 4: Herstellung einer Haarfärbecreme für Oxidationshaarfarben

80 kg Wasser wurden kalt (15 °C) in einem Rührkessel vorgelegt, an den ein Homogenisator vom Typ Becomix Duo-Homogenisator DH 2000 (Berents GmbH & Co. KG) angeschlossen war. Über den zusätzlichen Anschluß des Homogenisators wurden 8 kg 70%iges Natriumlaurylethersulfat in das vorgelegte Wasser eingetragen, wobei ein Vakuum angelegt wurde. Anschließend wurden die Farbstoffvorprodukte sowie die weiteren, üblichen hydrophilen Hilfsund Zusatzstoffe von oben unter Rühren zu dem Ansatz gegeben.

Eine Mischung aus 10,5 kg Glycerylstearat, 3,8 kg Lanolinalkohol, 0,85 kg Glykoldistearat und 30 kg Cetearylalkohol wurde bei 77 °C aufgeschmolzen und über den zusätzlichen Anschluß des Homogenisators eingetragen, wobei ein Vakuum von 0,3 bar angelegt wurde und die Umfangsgeschwindigkeit des Homoginsators 28 m/s betrug. Die Mischtemperatur betrug 37°C. Es entstand eine homogene, viskose Masse ohne Wachs- oder Farbstippen, die unmittelbar anschließend abgefüllt werden kann.

## Patentansprüche

1. Verfahren zur Herstellung von wäßrigen Emulsionen oder Suspensionen wobei
(A) in einem Behälter eine wäßrige Phase vorgelegt wird, wobei an den Behälter ein Homogenisator vom Rotor/Stator-Typ angeschlossen ist und der Homogenisator einen zusätzlichen Anschluß aufweist, durch den eine einzuhomogenisierende Phase direkt auf den Rotor aufgegeben werden kann und diese Phase erst in den Zahnkränzen des Homogenisators mit der vorgelegten Phase in Berührung kommt
(B) der Homogenisator gestartet wird und danach
(C) eine einzuhomogenisierende, in der vorgelegten Phase unlösliche zweite Phase, welche bei Raumtemperatur fest vorliegt, in geschmolzenem Zustand über den zusätzlichen Anschluß des Homogenisators zugeführt wird,
wobei die wäßrige Phase nicht erwärmt ist, das heißt bei Umgebungstemperatur oder darunter vorliegt.

2. Verfahren nach Anspruch 1, **dadurch gekennzeichnet, daß** es sich bei der einzuhomogenisierenden Phase um ein geschmolzenes Wachs handelt.

3. Verfahren zur Herstellung einer Suspension nach Anspruch 2, **dadurch gekennzeichnet, daß** das geschmolzene Wachs in nicht erwärmtes Wasser ohne Emulgator eingebracht wird.

4. Verfahren nach einem der vorstehenden Ansprüche, **dadurch gekennzeichnet, daß** vor Zuführung der einzuhomogenisierenden Phase ein Emulgator oder ein Tensid über den Homogenisator eingebracht wird.

5. Verfahren zur Herstellung einer Trübungsmittelzusammensetzung, wobei
(A) in einem Behälter nicht erwärmtes Wasser vorgelegt wird, wobei an den Behälter ein Homogenisator vom Rotor/Stator-Typ angeschlossen ist und der Homogenisator einen zusätzlichen Anschluß aufweist, durch den eine einzuhomogenisierende Phase direkt auf den Rotor aufgegeben werden kann und diese Phase erst in den Zahnkränzen des Homogenisators mit der vorgelegten Phase in Berührung kommt
(B) der Homogenisator gestartet wird,
(C) ein Alkylethersulfat über den zusätzlichen Anschluß des Homogenisators zugeführt und dadurch in dem Wasser gelöst wird und
(D) ein Trübungsmittel in geschmolzenem Zustand über den zusätzlichen Anschluß des Homogenisators zugeführt und dadurch in die Tensidlösung einhomogenisiert wird.

6. Verfahren zur Herstellung einer Haarfärbecreme, wobei
(A) in einem Behälter Haarfarbstoffe oder Farbstoffvorprodukte und gegebenenfalls weitere übliche Zusatzstoffe in nicht erwärmtem Wasser vorgelegt werden, wobei an den Behälter ein Homogenisator vom Rotor/Stator-Typ angeschlossen ist und der Homogenisator einen zusätzlichen Anschluß aufweist, durch den eine einzuhomogenisierende Phase direkt auf den Rotor aufgegeben werden kann und diese Phase erst in den Zahnkränzen des Homogenisators mit der vorgelegten Phase in Berührung kommt
(B) der Homogenisator gestartet wird,
(C) eine Schmelze einer bei Raumtemperatur wachsartigen Substanz mit gegebenenfalls darin gelösten weiteren, üblichen hydrophoben Zusatzstoffen über den zusätzlichen Anschluß des Homogenisators zugeführt und dadurch in die vorgelegte wäßrige Phase einhomogenisiert wird.

7. Verfahren zur Herstellung von kosmetischen oder pharmazeutischen Mitteln, wobei entweder die kosmetischen Wirkund Zusatzstoffe in der vorgelegten Phase enthalten sind und danach die Herstellung der Emulsion oder der Suspension nach einem der vorstehenden Ansprüche erfolgt, oder wobei zunächst die Emulsion oder Suspension nach einem der vorstehenden Ansprüche hergestellt wird und anschließend die kosmetischen oder pharmazeutischen Wirk- und Zusatzstoffe eingebracht werden.

8. Verfahren nach Anspruch 7, **dadurch gekennzeichnet, daß** zum Schluß durch Zugabe von verdickend wirkenden Stoffen die Endviskosität des Mittels eingestellt wird.

9. Verfahren nach einem der vorgenannten Ansprüche, **dadurch gekennzeichnet, daß** das Verhältnis des die einzuhomogenisierende Phase zuführenden Teilstroms zu dem die vorgelegte Phase zuführenden Teilstrom so gewählt ist, daß die einzuhomogenisierende Phase im wesentlichen nicht in einen den Zahnkränzen der Rotor/Statoreinheit vorgelagerten Bereich transportiert wird, wo sie vor Homogenisierung mit der vorgelegten Phase in Kontakt kommen kann.

10. Verfahren nach Anspruch 9, **dadurch gekennzeichnet, daß** der die einzuhomogenisierende Phase zuführende Teilstrom durch Anlegen eines Druckunterschieds eingestellt wird und der die vorgelegte Phase zuführende Teilstrom durch Einstellung der Umfangsgeschwindigkeit des Rotors eingestellt wird.

11. Verfahren nach einem der vorgenannten Ansprüche, **dadurch gekennzeichnet, daß** die einzuhomogenisierende Substanz eine bei Raumtemperatur feste oder wachsartige, hydrophobe Substanz ist, die in geschmolzenem Zustand zugeführt wird und wobei die Temperaturen von vorgelegter Phase und zugeführter Schmelze so gewählt sind, daß die resultierende Mischtemperatur unterhalb des Kristallisations- oder Erstarrungspunktes der einzuhomogenisierenden Substanz liegt.

12. Verwendung eines Homogenisators vom Rotor/Stator-Typ welcher einen zusätzlichen Anschluß aufweist, durch den eine einzuhomogenisierende Substanz direkt auf den Rotor aufgegeben werden kann und die Substanz erst in den Zahnkränzen des Homogenisators mit der externen, wäßrigen Phase in Berührung kommt, zur Herstellung von Suspensionen aus geschmolzenem Wachs oder geschmolzenen wachsartigen Stoffen und nicht erwärmtem, das heißt bei Umgebungstemperatur oder darunter vorliegendem Wasser.

13. Verwendung nach Anspruch 12 zur Herstellung von Lotionen, Cremes, Salben, emulsionsförmigen kosmetischen Mitteln, emulsionsförmigen Lebensmitteln oder emulsionsförmigen pharmazeutischen Mitteln.

14. Verwendung nach Anspruch 12 oder 13 zur Herstellung von Haarpflegemitteln, Haarfärbemitteln, Hautcremes, Soßen, Mayonnaisen, cremigen Brotaufstrichen, emulsionsförmigen Salatdressings oder Margarine.

15. Wäßrige Emulsion oder Suspension, hergestellt nach dem Verfahren gemäß einem der Ansprüche 1 bis 11.

## Claims

1. Method for producing aqueous emulsions or suspensions, where
(A) an aqueous phase is initially introduced into a container, where a homogenizer of the rotor/stator type is connected to the container and the homogenizer has an additional connection through which a phase to be homogenized in can be placed directly onto the rotor and this phase only comes into contact with the initially introduced phase in the toothed rims of the homogenizer
(B) the homogenizer is started and then
(C) a second phase, insoluble in the initially introduced phase, to be homogenized in and which is a solid at room temperature is introduced in the molten state via the additional connection of the homogenizer,
where the aqueous phase is not warmed, i.e. is at ambient temperature or below.

2. Method according to Claim 1, **characterized in that** the phase to be homogenized in is a molten wax.

3. Method for producing a suspension as claimed in Claim 2, **characterized in that** the molten wax is introduced into nonheated water without emulsifier.

4. Method according to one of the preceding claims, **characterized in that** prior to introducing the phase to be homogenized in, an emulsifier or a surfactant is introduced via the homogenizer.

5. Method for producing an opacifier composition, where
(A) nonheated water is initially introduced into a container, where a homogenizer of the rotor/stator type is connected to the container and the homogenizer has an additional connection through which a phase to be homogenized in can be placed directly onto the rotor and this phase only comes into contact with the initially introduced phase in the toothed rims of the homogenizer
(B) the homogenizer is started,
(C) an alkyl ether sulphate is introduced via the additional connection of the homogenizer and thereby dissolved in the water and
(D) an opacifier is introduced in the molten state via the additional connection of the homogenizer and thereby homogenized into the surfactant solution.

6. Method for producing a hair-colouring cream, where
(A) hair dyes or dye precursors and optionally further customary additives are initially introduced in nonheated water into a container, where a homogenizer of the rotor/stator type is connected to the container and the homogenizer has an additional connection through which a phase to be homogenized in can be placed directly onto the rotor and this phase only comes into contact with the initially introduced phase in the toothed rims of the homogenizer
(B) the homogenizer is started,
(C) a melt of a substance which is wax-like at room temperature optionally with further customary hydrophobic additives dissolved therein is introduced via the additional connection of the homogenizer and thereby is homogenized into the initially introduced aqueous phase.

7. Method of producing cosmetic or pharmaceutical compositions, where either the cosmetic active ingredients and additives are present in the initially introduced phase and then the emulsion or the suspension is produced in accordance with one of the above claims, or where firstly the emulsion or suspension is produced in accordance with one of the above claims and then the cosmetic or pharmaceutical active ingredients and additives are introduced.

8. Method according to Claim 7, **characterized in that**, the final viscosity of the composition is adjusted, by finally adding thickeners.

9. Method according to one of the abovementioned claims, **characterized in that** the ratio of the partial stream supplying the phase to be homogenized in to the partial stream supplying the initially introduced phase is chosen such that the phase to be homogenized in is essentially not transported into a zone located before the toothed rims of the rotor/stator unit where it can come into contact with the initially introduced phase before homogenization.

10. Method according to Claim 9, **characterized in that** the partial stream supplying the phase to be homogenized in is adjusted by applying a pressure differential and the partial stream supplying the initially introduced phase is adjusted by adjusting the peripheral speed of the rotor.

11. Method according to one of the abovementioned claims, **characterized in that** the substance to be homogenized in is a hydrophobic substance which is solid or wax-like at room temperature, which is introduced in the molten state and where the temperatures of initially introduced phase and supplied melt are chosen such that the resulting mixing temperature is below the crystallization or solidification point of the substance to be homogenized in.

12. Use of a homogenizer of the rotor/stator type which has an additional connection through which a substance to be homogenized in can be placed directly onto the rotor and the substance only comes into contact with the external aqueous phase in the toothed rims of the homogenizer, for producing suspensions of molten wax or molten wax-like substances and nonheated water, i.e. water at ambient temperature or below.

13. Use according to Claim 12 for producing lotions, creams, ointments, cosmetic compositions in the form of emulsions, foods in the form of emulsions or pharmaceutical compositions in the form of emulsions.

14. Use according to Claim 12 or 13 for producing hair care compositions, hair colorants, skin creams, sauces, mayonnaises, creamy sandwich spreads, salad dressings in the form of emulsions or margarines.

15. Aqueous emulsion or suspension produced by the method according to one of Claims 1 to 11.

## Revendications

1. Procédé pour la préparation d'émulsions ou de suspensions aqueuses, dans lequel
(A) on dispose au préalable une phase aqueuse dans un récipient, un homogénéisateur du type rotor/stator étant raccordé au récipient et l'homogénéisateur comportant un raccord supplémentaire par lequel une phase à incorporer de façon homogène peut être amenée directement sur le rotor et cette phase n'entrant en contact avec la phase disposée au préalable que dans les couronnes dentées de l'homogénéisateur,
(B) on met en marche l'homogénéisateur et ensuite
(C) on envoie à l'homogénéisateur, par le raccord supplémentaire, une seconde phase à incorporer de façon homogène, insoluble dans la phase disposée au préalable, à l'état fondu, qui est solide à la température ambiante,
la phase aqueuse n'étant pas chauffée, c'est-à-dire se trouvant à la température ambiante ou au-dessous.

2. Procédé selon la revendication 1, **caractérisé en ce que** la phase à incorporer de façon homogène consiste en une cire fondue.

3. Procédé pour la préparation d'une suspension selon la revendication 1, **caractérisé en ce que** la cire fondue est introduite dans de l'eau non chauffée, sans émulsifiant.

4. Procédé selon l'une quelconque des revendications précédentes, **caractérisé en ce qu'**avant l'addition de la phase à incorporer de façon homogène, on introduit un émulsifiant ou un tensioactif au moyen de l'homogénéisateur.

5. Procédé pour la préparation d'une composition d'opacifiant, dans lequel
(A) on dispose au préalable de l'eau non chauffée dans un récipient, un homogénéisateur du type rotor/stator étant raccordé au récipient et l'homogénéisateur comportant un raccord supplémentaire par lequel une phase à incorporer de façon homogène peut être amenée directement sur le rotor et cette phase n'entrant en contact avec la phase disposée au préalable que dans les couronnes dentées de l'homogénéisateur,
(B) on met en marche l'homogénéisateur,
(C) on envoie un alkyléthersulfate à l'homogénéisateur, par le raccord supplémentaire, et on le dissout ainsi dans l'eau, et
(D) on introduit dans l'homogénéisateur un opacifiant à l'état fondu, par le raccord supplémentaire, et on l'incorpore ainsi de façon homogène dans la solution de tensioactif.

6. Procédé pour la préparation d'une crème colorante pour cheveux dans lequel
(A) dans un récipient on dispose au préalable dans de l'eau non chauffée des colorants pour cheveux ou des précurseurs de colorants et éventuellement d'autres additifs usuels, un homogénéisateur du type rotor/stator étant raccordé au récipient et l'homogénéisateur comportant un raccord supplémentaire par lequel une phase à incorporer de façon homogène peut être amenée directement sur le rotor et cette phase n'entrant en contact avec la phase disposée au préalable que dans les couronnes dentées de l'homogénéisateur,
(B) on met en marche l'homogénéisateur,
(C) on envoie à l'homogénéisateur, par le raccord supplémentaire, une masse fondue d'une substance cireuse à la température ambiante, avec éventuellement d'autres additifs hydrophobes usuels dissous dans celle-ci, et on l'incorpore ainsi de façon homogène dans la phase aqueuse disposée au préalable.

7. Procédé pour la fabrication de produits cosmétiques ou pharmaceutiques, dans lequel soit les actifs et additifs cosmétiques sont contenus dans la phase disposée au préalable et ensuite la préparation de l'émulsion ou de la suspension s'effectue selon l'une quelconque des revendications précédentes, soit on prépare d'abord l'émulsion ou la suspension selon l'une quelconque des revendications précédentes et on introduit ensuite les actifs et additifs cosmétiques ou pharmaceutiques.

8. Procédé selon la revendication 7, **caractérisé en ce qu'**enfin on ajuste la viscosité finale du produit par addition de substances à effet épaississant.

9. Procédé selon l'une quelconque des revendications précédentes, **caractérisé en ce que** le rapport du courant partiel amenant la phase à incorporer de façon homogène au courant partiel amenant la phase disposée au préalable est choisi de manière que la phase à incorporer de façon homogène ne soit pratiquement pas transportée dans une zone placée avant les couronnes dentées de l'unité de rotor/stator, où elle peut entrer en contact avec la phase disposée au préalable avant l'homogénéisation.

10. Procédé selon la revendication 9, **caractérisé en ce que** le courant partiel amenant la phase à incorporer de façon homogène est réglé par application d'une différence de pression et le courant partiel amenant la phase disposée au préalable est réglé par ajustement de la vitesse périphérique du rotor.

11. Procédé selon l'une quelconque des revendications précédentes, **caractérisé en ce que** la substance à incorporer de façon homogène est une substance hydrophobe solide ou cireuse à la température ambiante, qui est amenée à l'état fondu, et les températures de la phase disposée au préalable et de la masse fondue amenée étant choisies de manière que la température résultante du mélange se situe au-dessous du point de cristallisation ou de solidification de la substance à incorporer de façon homogène.

12. Utilisation d'un homogénéisateur du type rotor/stator qui comporte un raccord supplémentaire par lequel une substance à incorporer de façon homogène peut être amenée directement sur le rotor et la substance n'entre en contact avec la phase aqueuse externe que dans les couronnes dentées de l'homogénéisateur, pour la préparation de suspensions à base de cire fondue ou de substances cireuses fondues et d'eau non chauffée, c'est-à-dire se trouvant à la température ambiante ou au-dessous.

13. Utilisation selon la revendication 12, pour la préparation de lotions, crèmes, pommades, produits cosmétiques sous forme d'émulsions, produits alimentaires sous forme d'émulsions ou produits pharmaceutiques sous forme d'émulsions.

14. Utilisation selon la revendication 12 ou 13, pour la fabrication de produits de soin capillaires, de produits de teinture pour cheveux, de crèmes pour la peau, de sauces, de mayonnaises, de pâtes crémeuses à tartiner, de sauces pour salades sous forme d'émulsions ou de margarines.

15. Emulsion ou suspension aqueuse, préparée selon le procédé conforme à l'une quelconque des revendications 1 à 11.
